# EUROPÄISCHE PATENTSCHRIFT

(11) **EP 0 630 609 B1**
(45) Veröffentlichungstag und Bekanntmachung des Hinweises auf die Patenterteilung: **04.10.2001**
(21) Anmeldenummer: 94109001.1
(22) Anmeldetag: 13.06.1994
(51) Int. Cl.: A61B 5/15

(54) **Blutlanzettenvorrichtung zur Entnahme von Blut für Diagnosezwecke**
Blood lancet device for obtaining blood samples for diagnosis purposes
Dispositif à lancette pour prélèvements sanguins a des fins de diagnostique

(30) Priorität: 21.06.1993 DE 4320463
(43) Veröffentlichungstag der Anmeldung: 28.12.1994
(73) Patentinhaber: Roche Diagnostics GmbH, 68305 Mannheim (DE)
(72) Erfinder: Lange, Hans, D-68623 Lampertheim (DE); Argauer, Herbert, D-92712 Pirk (DE)
(74) Vertreter: Pfeifer, Hans-Peter, Dr.

(56) Entgegenhaltungen:
- EP-A- 0 458 451
- EP-A- 0 565 970
- WO-A-93/19671
- GB-A- 1 085 141

## Beschreibung

Die Erfindung betrifft eine Blutlanzettenvorrichtung zur Entnahme von Blut für Diagnosezwecke, umfassend ein Gehäuse, einen in dem Gehäuse beweglichen Lanzettenhalter zur Halterung einer Lanzette, welche einen Lanzettenkörper aus Kunststoff und eine in dem Lanzettenkörper fixierte Nadel umfaßt und einen Lanzettenantrieb zum Antreiben der Einstich- und Rückführbewegung des Lanzettenhalters mit einer darin gehaltenen Lanzette, wobei das Gehäuse an seinem in Einstichrichtung vorderen Ende ein auswechselbares Hautkontaktteil mit einer Austrittsöffnung für die Lanzette aufweist, an dem eine Kontaktfläche zum Andruck an die Haut bei der Benutzung der Blutlanzettenvorrichtung vorgesehen ist und das Hautkontaktteil und die Lanzette Bestandteil eines zur einmaligen Verwendung vorgesehenen, in einem Handhabungsschritt am vorderen Ende des Gehäuses einsetzbaren, Disposables ist, das so ausgebildet ist, daß die Lanzette nur zusammen mit dem Hautkontaktteil in das Gehäuse eingesetzt werden kann.

Auch ein solches Disposable ist Gegenstand des Erfindung

Bei verschiedenen Krankheiten ist es notwendig, das menschliche Blut hinsichtlich bestimmter Blutwerte zu untersuchen. Oftmals ist es dabei ausreichend, durch Erzeugung einer kleinen Stichwunde dem Körper nur eine geringe Menge Blut in Form eines Blutstropfens zu entnehmen. Ein besonders wichtiger derartiger Fall ist die Diabetes, bei der das Blut in regelmäßigen Abständen auf den Glucosegehalt untersucht werden muß.

Zur Erzeugung der Stichwunde werden üblicherweise Blutlanzettenvorrichtungen verwendet, die aus einem Stechgerät und hierzu angepaßten auswechselbaren Lanzetten bestehen. Das Stechgerät enthält den Lanzettenhalter, in den jeweils eine Lanzette auswechselbar eingesetzt werden kann. Beim Einstichvorgang wird der Lanzettenhalter mit der Lanzette schnell in Einstichrichtung bewegt, bis die Lanzettenspitze aus der Austrittsöffnung des Hautkontaktteils austritt und eine kleine Stichwunde in dem Körperteil, gegen das das Hautkontaktteil gedrückt wird, erzeugt. Danach wird der Lanzettenhalter mit der Lanzette entgegen der Einstichrichtung zurückbewegt. Eine solche Lanzettenvorrichtung wird beispielsweise in dem US-Patent 4,442,836 beschrieben.

Im folgenden wird das Ende des Stechgerätes, an dem sich die Austrittsöffnung befindet, als vorderes Ende und das gegenüberliegende Ende als hinteres Ende bezeichnet.

Um Infektionen zu vermeiden, soll für jeden Einstichvorgang eine neue Lanzette verwendet werden. Das in der genannten US-Patentschrift beschriebene Gerät ist aus diesem Grund so ausgebildet, daß nach jedem Einstichvorgang beim erneuten Spannen des Gerätes die gebrauchte Lanzette automatisch aus dem Gerät ausgeworfen wird.

Eine Infektionsgefahr kann jedoch nicht nur von der Lanzette selbst, sondern auch von der gegen die Haut gedrückten Kontaktfläche des Stechgerätes ausgehen. Dies gilt vor allem im Krankenhausbereich und in ärztlichen Praxen, wo dasselbe Blutentnahmegerät für unterschiedliche Patienten verwendet wird. Hier besteht aufgrund der möglichen Übertragung von im Blut der Patienten enthaltenen Krankheitserregern eine große Infektionsgefahr, beispielsweise mit Aids- oder Hepatitis B-Viren.

Um das Risiko durch Infektionen über die Hautkontaktfläche auszuschließen, ist bei einem unter dem Handelnamen "Glucoletcombi" von der Firma Ames/USA vertriebenen vorbekannten Gerät der eingangs bezeichneten Art ein spezielles Hautkontaktteil vorgesehen, welches gemeinsam mit der Lanzette ausgewechselt wird. Dabei sind die Lanzette und das Hautkontaktteil zwei getrennte Teile, die zu einer gemeinsam am unteren Ende des Stechgerätes ansetzbaren, zur einmaligen Verwendung vorgesehenen Einheit zusammengefaßt sind. Eine solche zur einmaligen Verwendung vorgesehene Einheit aus Lanzette und Hautkontaktteil wird hier als Disposable bezeichnet.

Bei der vorbekannten Blutlanzettenvorrichtung ist das Hautkontaktteil als längliche Hülse ausgebildet, deren Länge fast ein Drittel der Gesamtlänge der Blutlanzettenvorrichtung ausmacht und ihr unteres Ende vollständig umgibt (wenn sie benutzungsbereit ist, d.h. sich im "Benutzungszustand" befindet). In der Hülse sitzt die Lanzette als separates Bauteil, wobei sie nach vorne hin durch elastisch federnde Kunststoffstreifen gehalten wird, die die Rückführbewegung nach dem Einstich bewirken. Das Spannen dieses Gerätes erfolgt durch manuelles Zurückschieben des Lanzettenhalters, der im Benutzungszustand von dem hülsenförmigen Hautkontaktteil abgedeckt wird. Nach dem Einstich kann deshalb das Gerät erst dann wieder gespannt werden, wenn das Hautkontaktteil zuvor abgenommen wurde. Dadurch wird zwar gewährleistet, daß das Disposable bestehend aus Hautkontaktteil und Lanzette entfernt werden muß, bevor die Vorrichtung gespannt oder neu benutzt wird. Es wird aber nicht sicher verhindert, daß nach der Abnahme des Disposables und dem erneuten Spannen der Blutlanzettenvorrichtung dasselbe Disposable nochmals in das Gehäuse eingesetzt und verwendet wird. Eine versehentliche oder eine beabsichtigte Wiederverwendung des Disposables (aufgrund von Unkenntnis über die damit verbundenen Gefahren) kann somit nicht ausgeschlossen werden.

Der Erfindung liegt daher die Aufgabe zugrunde, eine Wiederverwendung sämtlicher Teile einer Blutlanzettenvorrichtung, die mit dem Blut des Patienten in Kontakt kommen können, sicher zu verhindern.

Die Aufgabe wird durch eine Blutlanzettenvorrichtung nach Anspruch 1 und ein Disposable nach Anspruch 7 gelöst.

Charakteristisch für die Erfindung ist, daß das Disposable sich beim Einsetzen in das Stechgerät in einem Einsetzungszustand befindet, in dem das Hautkontaktteil (direkt oder indirekt) zu einer Einheit mit dem Lanzettenkörper verbunden ist. Dieser Einsetzungszustand wird beibehalten, bis sich entweder das Hautkontaktteil oder die Lanzette oder beide in ihrer Endlage (Benutzungsposition) in der Blutlanzettenvorrichtung befinden. In dem Einsetzungszustand ist die Blutlanzettenvorrichtung nicht benutzbar, weil der Lanzettenkörper fest mit dem Hautkontaktteil verbunden ist.

Durch Trennen der Sollbruchstelle wird das Disposable von dem Einsetzungszustand in einen Benutzungszustand überführt, in dem der Lanzettenkörper und damit die Lanzette frei ist, so daß die Einstich- und Rückführbewegung zur Erzeugung einer Stichwunde möglich ist. Das Hautkontaktteil und die Lanzette sind dabei so ausgebildet, daß sie im Benutzungszustand nach Trennung der Sollbruchstelle praktisch nicht (allenfalls unter Zuhilfenahme zusätzlicher Werkzeuge wie beispielsweise einer Pinzette) in das Stechgerät eingesetzt werden können. Insbesondere ist das Hautkontaktteil so klein, daß es praktisch nur in Verbindung mit dem Disposable am vorderen Ende der Blutlanzettenvorrichtung befestigt werden kann. Dadurch wird eine Wiederverwendung sowohl benutzter Lanzetten als auch benutzter Hautkontaktteile nach Trennung der Sollbruchstelle praktisch ausgeschlossen.

Die Spitze der Lanzette ist üblicherweise mit einer Spitzenschutzkappe geschützt, die über eine Sollbruchstelle mit dem Lanzettenkörper in Verbindung steht und vor der Benutzung der Lanzette abgenommen wird. Auch bei der Erfindung ist vorzugsweise eine solche Spitzenschutzkappe vorgesehen, wobei die Sollbruchstelle zwischen Spitzenschutzkappe und Lanzettenkörper eine zweite Sollbruchstelle (zusätzlich zu der ersten Sollbruchstelle an der Verbindung des Hautkontaktteils mit dem Lanzettenkörper) ist. Bei dieser Ausführungsform ist vorzugsweise das Disposable so ausgebildet, daß die erste und die zweite Sollbruchstelle in einem einzigen Handhabungsschritt (vorzugsweise gleichzeitig) trennbar sind. Hierdurch wird die Handhabung vereinfacht, weil mit einer einzigen Hand-bewegung das Disposable von dem Einsetzungszustand in den Benutzungszustand gebracht werden kann.

Die Sollbruchstelle kann mit dem Fachmann geläufigen Mitteln realisiert sein. Sie ist jedenfalls ein Steg oder Streifen von Material, das mechanisch schwächer als die übrige Konstruktion des Disposables ist, so daß die Teile des Disposables (Lanzettenkörper, Hautkontaktteil und gegebenenfalls Spitzenschutzkappe) gezielt an der mindestens einen Sollbruchstelle getrennt werden, wenn eine mechanische Belastung, beispielsweise durch Verdrehen der Teile gegeneinander um ihre Längsachse, ausgeübt wird.

Das gesamte Disposable ist vorzugsweise - mit Ausnahme der metallischen Lanzettennadel - ein Kunststoff-Spritzgußteil. Dabei können der Lanzettenkörper, das Hautkontaktteil und gegebenenfalls die Spitzenschutzkappe in einem Arbeitsgang im Kunststoff-Spritzgießverfahren hergestellt werden, wobei sich die Nadel in der Längsachse des Kunststoffteils befindet. Bei diesem Verfahren lassen sich Sollbruchstellen einfach als leicht trennbare Dünnstellen oder Stege des spritzgegossenen Kunststoffes realisieren.

Die Verbindung zwischen dem Hautkontaktteil und dem Lanzettenkörper muß nicht notwendigerweise direkt sein. Bei einer besonders bevorzugten Ausführungsform steht das Hautkontaktteil nur mittelbar über die Spitzenschutzkappe mit dem Lanzettenkörper in Verbindung, wobei sich die erste Sollbruchstelle an der Verbindungsstelle zwischen Hautkontaktteil und Spitzenschutzkappe (am Rand der in dem Hautkontaktteil vorgesehen Austrittsöffnung) befindet, während die zweite Sollbruchstelle wie üblich zwischen der Spitzenschutzkappe und dem Lanzettenkörper realisiert ist.

Ein erfindungsgemäßes Disposable kann kostengünstig hergestellt werden. Bei der Herstellung als Kunststoff-Spritzgußteil unterscheiden sich die Herstellungskosten kaum von denjenigen üblicherweise in Blutlanzettenvorrichtungen verwendeter Lanzetten. Ein geringer Kaufpreis ist ein zusätzlicher Anreiz zur Vermeidung der Wiederverwendung von benutzten Disposables. Da für die Handhabung des Disposables die ohnehin vorgesehene Spitzenschutzkappe benutzt werden kann, ist es ausreichend, wenn das Hautkontaktteil, bei dem beim Stand der Technik eine Hülse zur Handhabung vorgesehen ist, im wesentlichen als ringförmige Platte ausgebildet ist. Die Ausbildung des Hautkontaktteils als relativ kleines Bauteil führt neben einer weiteren Senkung der Herstellungskosten auch zu einer geringen Baugröße des gesamten Disposables.

Um eine Zwangsfolge der Betätigungsschritte der Blutlanzettenvorrichtung zu erreichen, kann bei einer bevorzugten Ausführungsform vorgesehen sein, daß der Lanzettenantrieb Mittel aufweist, durch die sichergestellt wird, daß ein wiederholtes Spannen des Lanzettenantriebs nur nach Entnahme der bei der vorhergehenden Einstich- und Rückführbewegung in der Lanzettenhalterung befindlichen Lanzette möglich ist. Unterschiedliche Möglichkeiten zur Realisierung dieser Funktion sind bekannt. In der eingangs erwähnten US-Patentschrift 4,442,836 ist beispielsweise die Lanzettenhalterung mit einem Hebelarm versehen, der beim Spannvorgang gegen einen ortsfesten Vorsprung stößt und dadurch die Lanzette zwangsläufig ausstößt. Dabei ist also das Spannen der Lanzettenvorrichtung nur bei gleichzeitigem Ausstoßen der benutzten Lanzette möglich. Bei dem ebenfalls erwähnten Gerät mit dem hülsenförmigen Hautkontaktteil wird eine Spannwiederholungssperre dadurch realisiert, daß das Zurückdrücken des Lanzettenhalters nur bei leerem Lanzettenhalter möglich ist. Eine besonders bevorzugte weitere Ausgestaltung wird weiter unten bei der Beschreibung eines Ausführungsbeispiels der Erfindung näher beschrieben.

Eine solche Ausführungsform ist besonders vorteilhaft, weil zusätzlich zu der erfindungsgemäßen Sicherung gegen die Wiederverwendung eines benutzten und dem Gerät entnommenen Hautkontaktteils und/oder einer benutzten und dem Gerät entnommenen Lanzette ("Wiederbenutzungssperre") sichergestellt wird, daß die noch im Gerät befindliche Lanzette kein zweites Mal verwendet werden kann. Damit ergibt sich ein praktisch vollständiger Schutz gegen Infektionsgefahren durch fahrlässig fehlerhafte Handhabung der Blutlanzettenvorrichtung.

Die Erfindung wird im folgenden anhand eines in den Figuren schematisch dargestellten Ausführungsbeispiels näher erläutert; es zeigen:
- Figur 1: eine Schnittdarstellung einer Blutlanzettenvorrichtung entlang ihrer Längsachse,
- Figur 2: eine teilweise aufgeschnittene Seitenansicht eines Disposables,
- Figur 3: eine Schnittdarstellung entlang der Linie III-III in Figur 2,
- Figur 4: eine Schnittdarstellung entlang der Linie IV-IV in Figur 2,
- Figur 5: eine Schnittdarstellung entlang der Linie V-V in Figur 1,
- Figur 6: eine Schnittdarstellung entlang der Linie VI-VI in Figur 1.

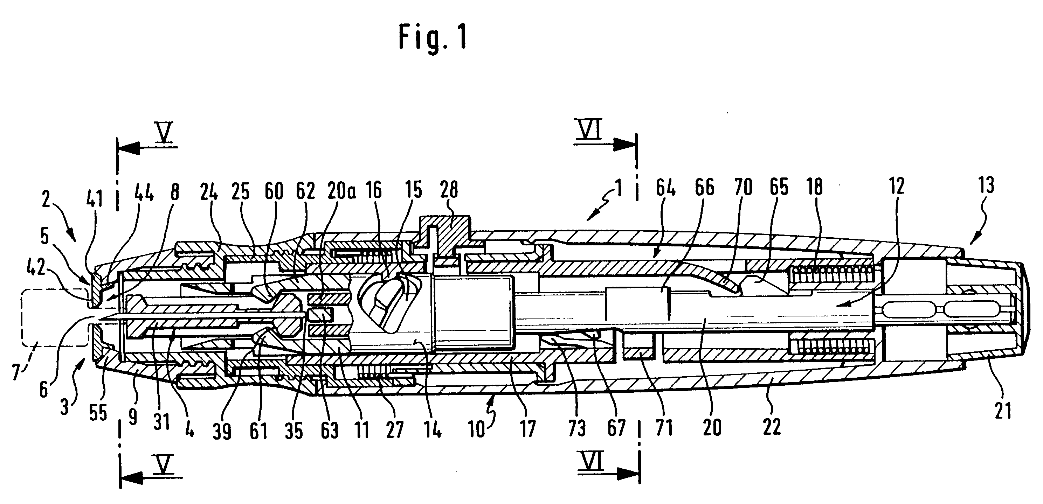

Figur 1 zeigt eine erfindungsgemäße Blutlanzettenvorrichtung 1 mit einem an ihrem vorderen Ende 2 eingesetzten Disposable 3. Das Disposable 3 besteht aus einer Lanzette 4, einem Hautkontaktteil 5 und einer Spitzenschutzkappe 7. Es ist in Figur 1 in seinem Benutzungszustand dargestellt, wobei die Lanzette 4 in einem Lanzettenhalter 11 sitzt und das Hautkontaktteil 5 so an einer Einstellkappe 9 des Gehäuses 10 befestigt ist, daß es dessen vordere Öffnung 8 - abgesehen von einer Austrittsöffnung 6 für die Lanzette - verschließt. Die Spitzenschutzkappe 7 ist im Benutzungszustand abgenommen und deswegen in Figur 1 gestrichelt dargestellt.

Die Einstich- und Rückführbewegung des Lanzettenhalters 11 und der darin befindlichen Lanzette 4 wird bei der dargestellten Ausführungsform durch eine Steuerkurve 15 gesteuert, die von einer nutenförmigen Ausnehmung in der kreisrunden Mantelfläche 14 des Lanzettenhalters 11 gebildet wird. In die Steuerkurve 15 greift ein Steuerzapfen 16 einer den Lanzettenhalter 11 in diesem Bereich umgebenden Antriebshülse 17 ein. Die Antriebshülse 17 erstreckt sich in dem Gehäuse 10 bis nahe an das in Einstichrichtung hintere Ende der Blutlanzettenvorrichtung 1. Hier ist auch eine die Antriebshülse 17 antreibende schraubenförmig gewundene Biegefeder 18 in der Antriebshülse 17 angeordnet. Durch die Biegefeder 18 verläuft ein mit seinem vorderen Ende 20a in den Lanzettenhalter 11 eingreifender Ausstoßer 20, an dessen hinterem Ende ein Betätigungsknopf 21 angeordnet ist. Die Antriebshülse 17 steht in Wirkverbindung mit einem Zwischenring 25, der mit einen Spannring 24 unlösbar verschraubt ist, auf den seinerseits die Einstellkappe 9 aufgeschraubt werden kann.

Zum Spannen der Blutlanzettenvorrichtung 1 wird das Gehäuse 10 an seinem oberen Hülsenteil 22 gehalten und der Spannring 24 mit dem Zwischenring 25 und damit die Antriebshülse 17 nach rechts (bei Blickrichtung entgegen der Einstichrichtung) gedreht. Diese Bewegung wird über die Antriebshülse 17 auf die Biegefeder 18 übertragen. Am Ende der Spannbewegung arretiert eine nicht dargestellte Arretierung die Antriebshülse, wodurch die Biegefeder 18 im gespannten Zustand bleibt. Eine Rückführfeder 27 dreht den Zwischenring 25 mit dem Spannring 24 durch eine Relativbewegung gegenüber der Antriebshülse 17 nach links in ihren Ausgangszustand zurück.

Der insgesamt mit 12 bezeichnete Lanzettenantrieb wird im wesentlichen durch die Biegefeder 18, die Antriebshülse 17, den Steuerzapfen 16, die Steuerkurve 15 und den Ausstoßer 20, der zugleich als Drehsicherung dient, gebildet. Wird durch ein Auslöseelement 28 der Lanzettenantrieb freigegeben, so führt die Antriebshülse 17 und der daran angeordnete Steuerzapfen 16 eine Linksdrehung (bei Blickrichtung entgegen der Einstichrichtung) aus. Dabei fährt der Steuerzapfen 16 die Steuerkurve 15 ab, wodurch der Lanzettenhalter 11 und die darin befindliche Lanzette 4 die Einstich- und Rückführbewegung ausführen.

Nähere Einzelheiten über den bei diesem Ausführungsbeispiel verwendeten Lanzettenantrieb können der Publikation der deutschen Patentanmeldung 42 12 315 entnommen werden.

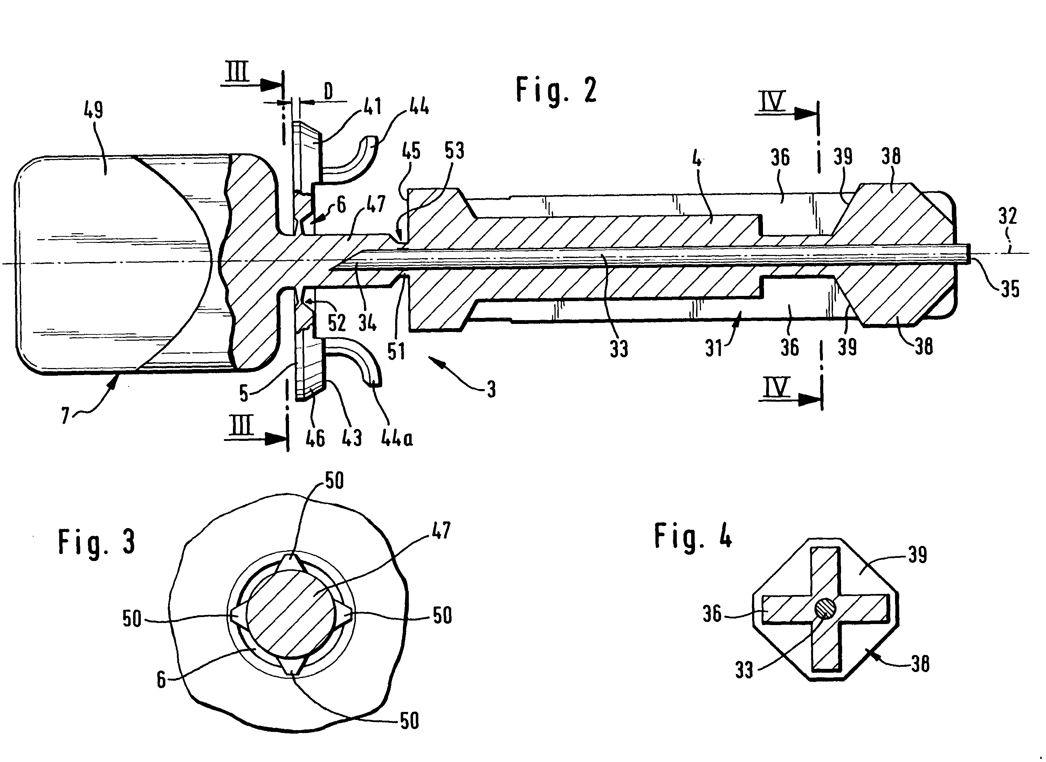

In Figur 2 ist das Disposable 3 in seinem Einsetzungszustand vor der Trennung seiner Bestandteile Lanzette 4, Hautkontaktteil 5 und Spitzenschutzkappe 7 dargestellt. Die Lanzette 4 weist einen Lanzettenkörper 31 aus Kunststoff auf, in dem entlang seiner gestrichelt dargestellten Längsachse 32 eine metallische Lanzettennadel 33 verläuft. Beide Enden der Lanzettennadel 33 ragen aus dem Lanzettenkörper 31 heraus. Das in Einstichrichtung vordere Ende der Lanzettennadel 33 ist zu einer Spitze 34 ausgebildet, während das hintere Ende eine Anschlagfläche 35 aufweist: Im Gegensatz zu sonst üblichen Lanzetten wird nicht der Lanzettenkörper, sondern die Anschlagfläche 35 der Lanzettennadel 33 zur exakten Positionierung der Lanzette 4 in dem Lanzettenhalter 11 verwendet. Dadurch sind exakt reproduzierbare Einstichtiefen bei aufeinanderfolgenden Einstichen mit unterschiedlichen Lanzetten, insbesondere bei geringen Einstichtiefen, erreichbar. Genauere Angaben zu diesem Aspekt können ebenfalls der deutschen Patentanmeldung 42 12 315 entnommen werden. Bei der hier dargestellten Lanzette hat der Lanzettenkörper vier sich entlang seiner gesamten Länge erstreckende gleiche Stege 36, die jeweils um die Längsachse 32 um 90° versetzt angeordnet sind, so daß sie im Querschnitt ein gleichschenkliches Kreuz bilden (Figur 4). In der Nähe des hinteren Endes des Lanzettenkörpers 31 befindet sich jeweils zwischen den Stegen 36 ein Zwischenkörper 38 mit geneigten Andruckflächen 39.

Das Hautkontaktteil 5 besteht im wesentlichen aus einem scheibenförmigen Ringprofilteil 41 und zwei daran angegossenen hakenförmigen Rastelementen 44, die zur Befestigung an der Einstellkappe 9 dienen. Die vordere Fläche des Hautkontaktteils 5 bildet die Kontaktfläche 42, mit der die Blutlanzettenvorrichtung 1 gegen die Haut gedrückt wird. Der Abstand der hakenförmig von der Längsachse 32 weggebogenen Rastelemente 44 zueinander ist größer als der Durchmesser der Stirnfläche 45 des Lanzettenkörpers 31. Im Zentrum des Ringprofilteils 41 befindet sich (in dem in Figur 1 dargestellten Benutzungszustand) die Austrittsöffnung 6 für die Lanzettenspitze 34.

Wie erwähnt sollte das Hautkontaktteil aus verschiedenen Gründen möglichst klein sein. Der Durchmesser ist höchstens so groß wie der Durchmesser des vorderen Ende 2 der Blutlanzettenvorrichtung 1. Die Dimension in Richtung der Längsachse 32 beträgt einschließlich der Befestigungselemente weniger als 1 cm, vorzugsweise weniger als 5 mm. Die entsprechende Dimension des im eingesetzten Zustand von außen zugänglichen Teils des Hautkontaktteils sollte noch erheblich geringer sein, um ein separates Einsetzen des Hautkontaktteils 5 praktisch unmöglich zu machen. Im dargestellten Ausführungsbeispiel ragt das Hautkontaktteil 5 nur um die in Figur 2 eingetragene Länge D vom vorderen Ende 2 der Blutlanzettenvorrichtung 1 hervor. Diese Dimension sollte allgemein weniger als 2 mm betragen. Im bevorzugten Anwendungsfall liegt sie bei weniger als 1 mm.

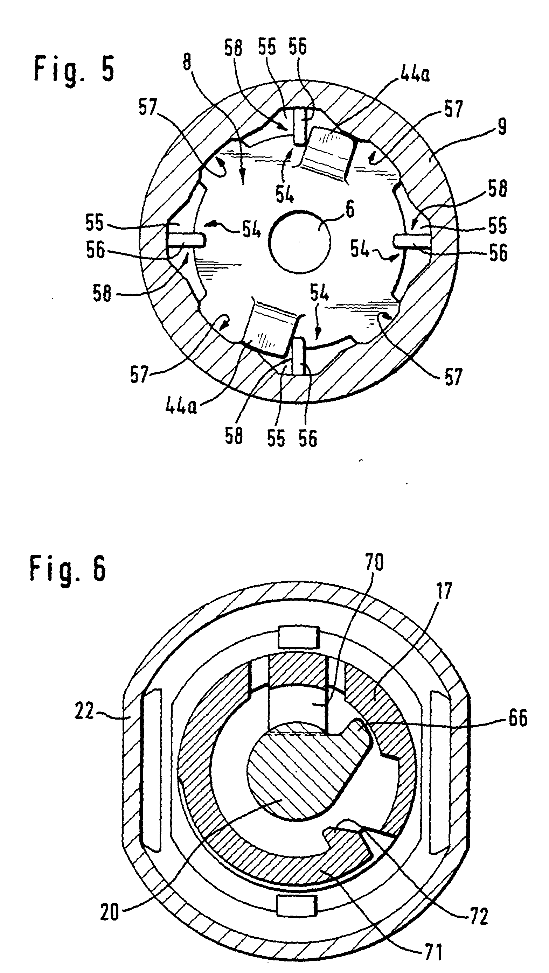

Wie in Figur 5 zu erkennen ist, weist die Einstellkappe 9 Befestigungsmittel 54 zur auswechselbaren Befestigung des Hautkontaktteils 5 auf. Im dargestellten Fall funktioniert die Befestigung nach Art eines Bajonettverschlusses. Entlang einer Umfangslinie der Öffnung 8 der Einstellkappe 9 erstrecken sich vier Absätze 55. Hinter jedem Absatz 55 ist jeweils mittig ein als Drehbegrenzungsanschlag 58 dienender Steg 56 angeordnet. Zwischen jeweils zwei Absätzen 55 befindet sich eine Einführausnehmung 57.

In Figur 2 ist zu sehen, daß sich die Spitzenschutzkappe 7 mit einem Schaft 47 durch die Austrittsöffnung 6 hindurch erstreckt und in dem vor der Austrittsöffnung 6 liegenden Abschnitt eine Griffzone 49 zur Handhabung des Disposables 3 aufweist. Der Durchmesser der Austrittsöffnung 6 sollte kleiner als 3,5 mm sein und vorzugsweise etwa 2 bis 2,8 mm betragen.

Die Spitzenschutzkappe 7 ist durch vier auf der Begrenzungsfläche der Austrittsöffnung 6 gleichmäßig verteilte Stege 50 mit dem Hautkontaktteil 5 verbunden (Figur 3). Die Stege 50 sind so ausgebildet, daß sie bei einer Drehbewegung der Spitzenschutzkappe 7 brechen und somit die erste Sollbruchstelle 52 bilden. Die Verbindung der Spitzenschutzkappe 7 mit dem Lanzettenkörper 31 wird von einem dünnwandigen umlaufenden Steg 51 zwischen der Stirnfläche 45 des Lanzettenkörpers 31 und dem Schaft 47 der Spitzenschutzkappe 7 gebildet. Dieser Steg 51 bildet die zweite Sollbruchstelle 53.

Der in Fig. 1 dargestellte Lanzettenhalter 11 hat zur Aufnahme der Lanzette 4 eine im Querschnitt im wesentlichen quadratische Aufnahmeausnehmung. Die quadratische Aufnahmeausnehmung ist auf die Stege 36 (Figur 4) des Lanzettenkörpers 31 so abgestimmt, daß der Lanzettenkörper 31 in vier unterschiedlichen, bezüglich der Längsachse 32 jeweils um 90° gedrehten Positionen, in den Lanzettenhalter 11 einführbar ist. In diesen vier Positionen ist auch das Hautkontaktteil 5 mit den Rastelementen 44 in die Einführausnehmungen 57 (Figur 5) der Einstellkappe 9 einführbar. In die Aufnahmeausnehmung des Lanzettenhalters 11 ragen zwei mit Schrägen 60 versehene Nasen 61, die sich jeweils an federelastisch auslenkbaren Spannzungen 62 befinden. Außerdem ist in der Aufnahmeausnehmung von den Nasen 61 aus entgegen der Einstichrichtung gesehen ein quer zur Einstechrichtung verlaufender Anschlagsteg 63 vorhanden.

Zum Einsetzen des Disposables 3 wird bei gespanntem Lanzettenantrieb 12 die Lanzette 4 in den Lanzettenhalter 11 gegen den Druck der Spannzungen 62 eingeführt, wodurch der Ausstoßer 20 nach hinten geschoben wird. Dabei gleiten die Nasen 61 auf den jeweils gegenüberliegenden Zwischenkörpern 38 des Lanzettenkörpers 31. Gegen Ende der Einführbewegung gelangen dann die beiden Nasen 61 jeweils mit ihrer Schräge 60 auf die Andruckfläche 39, wodurch das Disposable 3 in den Lanzettenhalter 11 hineingezogen wird. Die Einführbewegung ist beendet, wenn die Anschlagfläche 35 den Anschlagsteg 63 berührt. Gleichzeitig werden die beiden Rastelemente 44 durch zwei der Einführausnehmungen 57 in die Einstellkappe 9 eingeführt, so daß sich die hakenförmigen Enden 44a der beiden Rastelemente 44 etwa auf gleicher Höhe mit den Anschlagstegen 56 befinden.

In dieser Position ist das Disposable 3 in die Blutlanzettenvorrichtung 1 eingesetzt. Allgemein ist das Einsetzen des Disposables im Sinne der Erfindung dann beendet, wenn von den beiden Hauptbestandteilen des Disposables, nämlich der Lanzette 4 und dem Hautkontaktteil 5, mindestens eines seine Endposition in Richtung der Einstichbewegung erreicht hat. Bei der dargestellten Ausführungsform ist dies die Lanzette 4, die sich in ihrer Benutzungsposition in dem Lanzettenhalter 11 befindet.

Da im Einsetzungszustand die Sollbruchstellen noch intakt sind, ist die Blutlanzettenvorrichtung 1 noch nicht benutzbar. Um sie in den Benutzungszustand zu überführen, muß zuerst die zweite Sollbruchstelle zwischen dem Lanzettenkörper 31 und der Spitzenschutzkappe 7 durch eine Links- oder Rechtsdrehung der Spitzenschutzkappe 7 durchtrennt werden. Durch Weiterdrehen der Spitzenschutzkappe 7 in die vorher eingeschlagene Richtung kommen die Rastelemente 44 zum Anschlag gegen die jeweiligen Anschlagstege 56. In dieser Position hintergreifen die Enden 44a der Rastelemente 44 jeweils einen Absatz 55, so daß dadurch und durch Anliegen der konischen Umfangsfläche 46 des Ringprofilteils 41 an einer dazu kongruenten Fläche der Einstellkappe 9 eine sichere axiale und radiale Positionierung des Hautkontaktteils 5 gewährleistet ist. Eine Fortsetzung der Drehbewegung der Spitzenschutzkappe 7 führt zum Bruch der vier Stege 50, wodurch auch die erste Sollbruchstelle 52 getrennt ist.

Die beiden Sollbruchstellen wurden somit durch einen einzigen Handhabungsschritt, nämlich das Drehen der Spitzenschutzkappe 7, getrennt, wodurch die Blutlanzettenvorrichtung 1 vom Einsetzungszustand in den Benutzungszustand überführt wurde. Die einzelnen Elemente des Disposables 3 können im getrennten Zustand nicht (bzw. nur mit Hilfe eines Werkzeugs und mit erheblichem Aufwand, der eine unbeabsichtigte Fehlbenutzung ausschließt) wieder eingesetzt werden.

Bei der dargestellten bevorzugten Ausführungsform sind Mittel 64 (Figur 1) vorgesehen, durch die sichergestellt wird, daß ein wiederholtes Spannen des Lanzettenantriebs nur nach Entnahme der bei der vorhergehenden Einstichund Rückführbewegung in der Lanzettenhalterung befindlichen Lanzette möglich ist. Diese werden nachfolgend beschrieben.

Der Ausstoßer 20 ist auf seiner Mantelfläche mit einer in Einstichrichtung rampenartig ansteigenden Ausstoßsicherungsnase 65 versehen. Weiter vorne und gegenüber der Ausstoßsicherungsnase 65 am Umfang versetzt, hat der Ausstoßer 20 zudem einen Zapfen 66 (Fig. 1 und Fig. 6). Vor dem Zapfen 66 und gegenüber diesem und der Ausstoßsicherungsnase 65 am Umfang des Ausstoßers 20 versetzt, befindet sich auf der Mantelfläche des Ausstoßers 20 eine entgegen der Einstichrichtung rampenartig ansteigende Rückschiebe-Sicherungsnase 67. Das vordere Ende 20a des Ausstoßers 20 ist in der Weise gabelartig ausgebildet, daß es im Bereich des Anschlagsteges 63 im Lanzettenhalter 11 axial verschiebbar ist.

In Fig. 1 ist zu erkennen, daß die Antriebshülse 17 in der Nähe ihres hinteren Endes eine zunächst achsparallel verlaufende, dann leicht nach innen gekrümmte federelastische erste Schnappzunge 70 aufweist, die in die Antriebshülse 17 hineinragt. In Einstichrichtung weiter vorne weist die Antriebshülse 17 eine bogenförmig etwa entlang einer Umfangslinie verlaufende zweite federelastische Schnappzunge 71 auf. Das freie Ende der zweiten Schnappzunge 71 ist mit einer in das Innere der Antriebshülse 17 hineinragenden Nase 72 versehen, die gegenüber der ersten Schnappzunge 70 (Figur 6) am Umfang versetzt ist. Vor der zweiten Schnappzunge 71 ist eine weitere axial ausgerichtete federelastische dritte Schnappzunge 73 an der Antriebshülse 17 vorhanden. Diese sitzt auf der Innenfläche der Antriebshülse und bildet eine auslenkbare in Einstichrichtung ansteigende Rampe.

Die Antriebshülse 17 ist nun so auf den Ausstoßer 20 abgestimmt, daß sich bei einer Einstich- und Rückführbewegung der Lanzette 4 der Zapfen 66 des Ausstoßers 20 und die zweite Schnappzunge 71, wie in Fig. 1 und Fig. 6 dargestellt, auf gleicher Höhe befinden. Diese Bewegung der Lanzette 4 wird durch eine Linksdrehung der Antriebshülse 17 (in der Darstellung der Figur 6) bewirkt. Dabei rutscht die Nase 72 der zweiten Schnappzunge 71 über den Zapfen 66 und rastet dahinter ein.

Da der Ausstoßer 20 gegen Drehbewegungen gesichert ist, ist die zum Spannen erforderliche Rechtsdrehung der Antriebshülse 17 nicht möglich. Es muß daher zuerst das Disposable 3 durch Betätigen des Ausstoßers 20 in seinen Einzelteilen ausgestoßen werden. Dabei drückt das gabelartige vordere Ende 20a des Ausstoßers 20 durch die axiale Längsverschiebung die Lanzette 4 aus dem Lanzettenhalter 11. Der Lanzettenkörper 31 wiederum drückt mit seiner Stirnfläche 45 zwischen den Rastelementen 44 auf das Ringprofilteil 41. Dadurch rutschen die hakenförmigen Enden der Rastelemente 44 über die Absätze 55. Anschließend wird das Hautkontaktteil 5 und die Lanzette 4 ausgestoßen.

Durch diese axiale Verschiebung des Ausstoßers 20 gibt der Zapfen 66 nun die zweite Schnappzunge 71 frei. Eine Rechtsdrehung der Antriebshülse 17 und damit ein Spannen der Blutlanzettenvorrichtung 1 ist nun möglich. Bei der Ausstoßbewegung des Ausstoßers 20 ist aber auch die Rückschiebe-Sicherungsnase 67 über die dritte Schnappzunge 73 gerutscht und hinter ihr verrastet. Damit ist das zum Einsetzen eines Disposables 3 erforderliche Rückschieben des Ausstoßers 20 nicht möglich. Zwangsläufig muß zunächst die Blutlanzettenvorrichtung 1 durch eine Rechtsdrehung der Antriebshülse 17 gespannt werden. Dadurch wird auch die Rückschiebe-Sicherungsnase 67 gegenüber der Schnappzunge 73 gedreht und somit freigegeben.

Als nächstes folgt nun das Einführen des Disposables 3, wodurch der Ausstoßer 20 durch Andruck des Lanzettenkörpers 31 gegen das vordere Ende des Ausstoßers 20 zurückgeschoben wird. Bei diesem Vorgang bewegt sich die Ausstoßsicherungsnase 65 durch Auslenken der ersten Schnappzunge 70 unter dieser hinweg. Dabei verrastet die Ausstoßsicherungsnase 65 mit der Schnappzunge 70 sobald das Disposable seinen Einsetzungszustand in der Blutlanzettenvorrichtung 1 erreicht hat. Die Verrastung zwischen der Schnappzunge 70 und der Ausstoßsicherungsnase 65 verhindert ein (beispielsweise ungewolltes) Ausstoßen des Disposables 3, bevor es in den Benutzungszustand überführt wurde und die Lanzette die Einstich- und Rückführbewegung ausgeführt hat.

Damit ergibt sich insgesamt eine vollständige Zwangsführung der Betätigungsschritte Spannen, Disposable einsetzen, Sollbruchstellen trennen, Stechvorgang auslösen und Lanzette ausstoßen.

## Patentansprüche

1. Blutlanzettenvorrichtung (1) zur Entnahme von Blut für Diagnosezwecke, umfassend
ein Gehäuse (10),
einen in dem Gehäuse (10) beweglichen Lanzettenhalter (11) zur Halterung einer Lanzette (4), welche einen Lanzettenkörper (31) aus Kunststoff und eine in dem Lanzettenkörper (31) fixierte Lanzettennadel (33) umfaßt
und
einen Lanzettenantrieb (12) zum Antreiben der Einstich- und Rückführbewegung des Lanzettenhalters (11) mit der darin gehaltenen Lanzette (4),
wobei
das Gehäuse (10) an seinem in Einstichrichtung vorderen Ende (2) ein auswechselbares Hautkontaktteil (5) mit einer Austrittsöffnung (6) für die Lanzette (4) aufweist, an dem eine Kontaktfläche (42) zum Andruck an die Haut bei der Benutzung der Blutlanzettenvorrichtung (1) vorgesehen ist und
das Hautkontaktteil (5) und die Lanzette (4) Bestandteil eines einmalig verwendbaren, in einem Handhabungsschritt am vorderen Ende (2) des Gehäuses (10) einsetzbaren, Disposables (3) ist, das so ausgebildet ist, daß die Lanzette (4) nur zusammen mit dem Hautkontaktteil (5) eingesetzt werden kann,
**dadurch gekennzeichnet, daß**
in dem Disposable (3) das Hautkontaktteil (5) und der Lanzettenkörper (31) zu einer Einheit miteinander verbunden sind und an der Verbindung zwischen Hautkontaktteil (5) und Lanzettenkörper (31) eine erste Sollbruchstelle (52) vorgesehen ist, durch die das Hautkontaktteil (5) und der Lanzettenkörper (31) nach dem Einsetzen des Disposables (3) voneinander trennbar sind.

2. Blutlanzettenvorrichtung nach Anspruch 1, **dadurch gekennzeichnet, daß** die Lanzette (4) eine Spitzenschutzkappe (7) aus Kunststoff aufweist, welche mit dem Lanzettenkörper (31) über eine zweite Sollbruchstelle (53) verbunden ist und beide Sollbruchstellen nach dem Einsetzen des Disposables (3) trennbar sind.

3. Blutlanzettenvorrichtung nach Anspruch 2, **dadurch gekennzeichnet, daß** das Disposable (3) so ausgebildet ist, **daß** die erste Sollbruchstelle (52) und die zweite Sollbruchstelle (53) in einem einzigen Handhabungsschritt trennbar sind.

4. Blutlanzettenvorrichtung nach Anspruch 3, **dadurch gekennzeichnet, daß** die Lanzette (4) in dem Lanzettenhalter (11) nach dem Einsetzen des Disposables (3) drehfest gelagert ist und am vorderen Ende (2) des Gehäuses (10) ein Drehbegrenzungsanschlag (58) für das Hautkontaktteil (5) vorgesehen ist, wobei beide Sollbruchstellen durch Drehen der Spitzenschutzkappe (7) relativ zu der Lanzette (4) und dem Hautkontaktteil (5) trennbar sind.

5. Blutlanzettenvorrichtung nach einem der vorhergehenden Ansprüche, **dadurch gekennzeichnet, daß** der Lanzettenantrieb (12) Mittel (64) aufweist, durch die sichergestellt wird, **daß** ein wiederholtes Spannen des Lanzettenantriebs (12) nur nach Entnahme einer bei der vorhergehenden Einstich- und Rückführbewegung in dem Lanzettenhalter (11) befindlichen Lanzette (4) möglich ist.

6. Blutlanzettenvorrichtung nach einem der vorhergehenden Ansprüche, **dadurch gekennzeichnet, daß** das Hautkontaktteil (5) als Ringprofilteil (41) ausgebildet ist, welches im wesentlichen nur den vorderen Abschluß des Gehäuses (10) bildet.

7. Disposable zur einmaligen Verwendung mit einer Blutlanzettenvorrichtung (1) mit
einer Lanzette (4), welche einen Lanzettenkörper (31) aus Kunststoff und eine in dem Lanzettenkörper (31) fixierte Lanzettennadel (33) umfaßt und
einem an dem in Einstichrichtung vorderen Ende der Blutlanzettenvorrichtung auswechselbar befestigbaren Hautkontaktteil (5) mit einer Austrittsöffnung (6) für die Lanzette (4) und einer Kontaktfläche (42) zum Andruck an die Haut bei der Benutzung der Blutlanzettenvorrichtung (1),
wobei das Disposable so ausgebildet ist, daß die Lanzette (4) nur zusammen mit dem Hautkontaktteil (5) in einem Handhabungsschritt am vorderen Ende des Gehäuses (10) der Blutlanzettenvorrichtung (1) einsetzbar ist,
**dadurch gekennzeichnet, daß**
in dem Disposable (3) das Hautkontaktteil (5) und der Lanzettenkörper (31) zu einer Einheit miteinander verbunden sind und an der Verbindung zwischen Hautkontaktteil (5) und Lanzettenkörper (31) eine erste Sollbruchstelle (52) vorgesehen ist, durch die das Hautkontaktteil (5) und der Lanzettenkörper (31) nach dem Einsetzen des Disposables (3) voneinander trennbar sind.

8. Disposable nach Anspruch 7, **dadurch gekennzeichnet, daß** die Lanzette (4) eine Spitzenschutzkappe (7) aus Kunststoff aufweist, welche mit dem Lanzettenkörper (31) über eine zweite Sollbruchstelle (53) verbunden ist und beide Sollbruchstellen nach dem Einsetzen des Disposables (3) trennbar sind.

9. Disposable nach einem der Ansprüche 7 oder 8, **dadurch gekennzeichnet, daß** es so ausgebildet ist, **daß** die erste Sollbruchstelle (52) und die zweite Sollbruchstelle (53) in einem einzigen Handhabungsschritt trennbar sind.

10. Disposable nach Anspruch 9, daß die Spitzenschutzkappe (7) mit dem Hautkontaktteil (5) am Rand der Austrittsöffnung (6) verbunden und die erste Sollbruchstelle (52) an dieser Verbindung vorgesehen ist.

11. Disposable nach einem der Ansprüche 7 bis 10, **dadurch gekennzeichnet, daß** das Hautkontaktteil (5) in Einstichrichtung vor dem Lanzettenkörper (31) angeordnet ist und sich die Spitzenschutzkappe (7) durch die Austrittsöffnung (6) des Hautkontaktteils (5) hindurch erstreckt.

12. Disposable nach einem der Ansprüche 7 bis 11, **dadurch gekennzeichnet, daß** es, mit Ausnahme der metallischen Lanzettennadel, ein Kunststoff-Spritzgußteil ist.

## Claims

1. Blood lancet device (1) for withdrawing blood for diagnostic purposes, comprising
a housing (10),
a lancet holder (11), movable within the housing (10), for holding a lancet (4) which comprises a lancet body (31) made of a plastics material and a lancet needle (33) fixed in the lancet body (31)
and
a lancet drive (12) to drive the puncturing and retraction movement of the lancet holder (11) with a lancet (4) held therein,
in which
the housing (10) has, at its anterior end (2) in the puncturing direction, an exchangeable skin-contact part (5) with an outlet opening (6) for the lancet (4), said skin-contact part (5) being provided with a contact surface (42) for pressing against the skin when using the blood lancet device (1), and the skin-contact part (5) and lancet (4) form components of a disposable item (3) for once-only use which can be inserted at the anterior end (2) of the housing (10) in a single handling operation, said disposable item (3) being designed in such a way that the lancet (4) can be inserted only together with the skin-contact part (5),
**characterized in that**
in the disposable item (3), the skin-contact part (5) and the lancet body (31) are connected to one another forming an unit and at the point of connection between the skin-contact part (5) and the lancet body (31) a first predetermined breaking section (52) is provided by means of which the skin-contact part (5) and the lancet body (31) are separable from one another following insertion of the disposable item (3).

2. Blood lancet device according to claim 1, **characterized in that** the lancet (4) has a tip-protection cap (7) made of a plastics material, which is connected with the lancet body (31) via a second predetermined breaking section (53), and both predetermined breaking sections are separable following insertion of the disposable item (3).

3. Blood lancet device according to claim 2, **characterized in that** the disposable item (3) is designed in such a way that the first predetermined breaking section (52) and the second predetermined breaking section (53) are separable in a single handling operation.

4. Blood lancet device according to claim 3, **characterized in that,** following insertion of the disposable item (3), the lancet (4) in the lancet holder (11) is non-rotatably mounted and a rotation-limiting stop (58) for the skin-contact part (5) is provided at the anterior end (2) of the housing (10), both predetermined breaking sections being separable by rotation of the tip-protection cap (7) relative to the lancet (4) and the skin-contact part (5).

5. Blood lancet device according to any one of the preceding claims, **characterized in that** the lancet drive (12) has means (64) for ensuring that repeated tensioning of the lancet drive (12) is possible only after removal of a lancet (4) which during the preceding puncturing and retraction movement was located in the lancet holder (11).

6. Blood lancet device according to any one of the preceding claims, **characterized in that** the skin-contact part (5) is designed as a ring-shaped part (41), which essentially forms only the anterior face of the housing (10).

7. Disposable item for once-only use with a blood lancet device (1) comprising
a lancet (4) which comprises a lancet body (31) made of a plastics material and a lancet needle (33) fixed in the lancet body (31) and,
located at the anterior end of the blood lancet device in its puncturing direction, an exchangeable skin-contact part (5) with an outlet opening (6) for the lancet (4) and a contact surface (42) for pressing against the skin when using the blood lancet device (1),
said disposable item being designed in such a way that the lancet (4) can be inserted only together with the skin-contact part (5) in a single handling operation at the anterior end of the housing (10) of the blood lancet device (1),
**characterized in that**
in the disposable item (3), the skin-contact part (5) and the lancet body (31) are connected to one another forming an unit and at the point of connection between the skin-contact part (5) and the lancet body (31) a first predetermined breaking section (52) is provided by means of which the skin-contact part (5) and the lancet body (31) are separable from one another following insertion of the disposable item (3).

8. Disposable item according to claim 7, **characterized in that** the lancet (4) has a tip-protection cap (7) made of a plastics material, which is connected with the lancet body (31) via a second predetermined breaking section (53), and both predetermined breaking sections are separable following insertion of the disposable item (3).

9. Disposable item according to any one of claims 7 or 8, **characterized in that** it is designed in such a way that the first predetermined breaking section (52) and the second predetermined breaking section (53) are separable in a single handling operation.

10. Disposable item according to claim 9, **characterized in that** the tip-protection cap (7) is connected with the skin-contact part (5) at the edge of the outlet opening (6) and the first predetermined breaking section (52) is provided at this connection.

11. Disposable item according to any one of claims 7 to 10, **characterized in that** the skin-contact part (5) is disposed in front of the lancet body (31) in the puncturing direction, and the tip-protection cap (7) extends through the outlet opening (6) of the skin-contact part (5).

12. Disposable item according to any one of claims 7 to 11, **characterized in that** it is, with the exception of the metallic lancet needle, a plastics injection moulding.

## Revendications

1. Dispositif à lancette (1) pour prélèvements sanguins à des fins de diagnostique, comprenant
un boîtier (10),
un porte-lancette (11) mobile dans le boîtier (10), destiné à tenir une lancette (4) laquelle comprend un corps de lancette (31) en matière plastique et une aiguille de lancette (33) fixée dans le corps de lancette (31),
et
une commande de lancette (12) pour commander le mouvement d'introduction et de recul du porte-lancette (11) avec la lancette (4) qui y est tenue,
le boîtier (10), à son extrémité avant (2) dans le sens d'introduction, comprenant une pièce de contact cutané (5) échangeable muni d'un orifice de sortie (6) pour la lancette (4), sur laquelle pièce est prévue une surface de contact (42) destinée à s'appuyer contre la peau lors de l'utilisation du dispositif à lancette (1), et
la pièce de contact cutané (5) et la lancette (4) faisant partie d'un élément jetable (3) à usage unique pouvant être mis en place en une seule manipulation à l'extrémité avant (2) du boîtier (10), lequel élément est réalisé de telle façon que la lancette (4) ne peut être mise en place que conjointement avec la pièce de contact cutané (5),
**caractérisé en ce que**
dans l'élément jetable (3), la pièce de contact cutané (5) et le corps de lancette (31) sont reliés entre eux en formant une unité et **en ce qu'**au niveau de la liaison entre la pièce de contact cutané (5) et le corps de lancette (31), un premier point destiné à la rupture (52) est prévu grâce auquel la piècé de contact cutané (5) et le corps de lancette (31) sont séparables l'un de l'autre après la mise en place de l'élément jetable (3).

2. Dispositif à lancette selon la revendication 1, **caractérisé en ce que** la lancette (4) comprend un capuchon protecteur de pointe (7) en matière plastique, lequel est relié au corps de lancette (31) par un deuxième point destiné à la rupture (53) et **en ce que** les deux points destinés à la rupture sont séparables après la mise en place de l'élément jetable (3).

3. Dispositif à lancette selon la revendication 2, **caractérisé en ce que** l'élément jetable (3) est réalisé de telle façon que le premier point destiné à la rupture (52) et le deuxième point destiné à la rupture (53) sont séparables en une seule manipulation.

4. Dispositif à lancette selon la revendication 3, **caractérisé en ce que** la lancette (4), après la mise en place de l'élément jetable (3), est logée sans rotation possible dans le porte-lancette (11) et **en ce qu'**à l'extrémité avant (2) du boîtier (10), une butée de limitation de rotation (58) pour la pièce de contact cutané (5) est prévue, les deux points destinés à la rupture étant séparables en tournant le capuchon protecteur de pointe (7) par rapport à la lancette (4) et la pièce de contact cutané (5).

5. Dispositif à lancette selon l'une des revendications précédentes, **caractérisé en ce que** la commande de lancette (12) comprend des moyens (64) permettant d'assurer qu'une tension répétée de la commande de lancette (12) n'est possible qu'après avoir retiré une lancette (4) se trouvant dans le porte-lancette (11) lors du précédent mouvement d'introduction et de recul.

6. Dispositif à lancette selon l'une des revendications précédentes, **caractérisé en ce que** la pièce de contact cutané (5) est réalisée comme profilé annulaire (41) lequel ne constitue pour l'essentiel que la terminaison avant du boîtier (10).

7. Elément jetable à usage unique pourvu d'un dispositif à lancette (1) comprenant
une lancette (4), laquelle comprend un corps de lancette (31) en matière plastique et une aiguille de lancette (33) fixée dans le corps de lancette (31), et
une pièce de contact cutané (5) apte à être fixée de manière échangeable à l'extrémité avant dans le sens d'introduction du dispositif à lancette et pourvue d'un orifice de sortie (6) pour la lancette (4) et d'une surface de contact (42) destinée à s'appuyer contre la peau lors de l'utilisation du dispositif à lancette (1),
ledit élément jetable étant réalisé de telle façon que la lancette (4) ne peut être mise en place que conjointement avec la pièce de contact cutané (5), en une manipulation, à l'extrémité avant du boîtier (10) du dispositif à lancette (1),
**caractérisé en ce que**
dans l'élément jetable (3), la pièce de contact cutané (5) et le corps de lancette (31) sont reliés entre eux en formant une unité et **en ce qu'**au niveau de la liaison entre la pièce de contact cutané (5) et le corps de lancette (31), un premier point destiné à la rupture (52) est prévu grâce auquel la pièce de contact cutané (5) et le corps de lancette (31) sont séparables l'un de l'autre après la mise en place de l'élément jetable (3).

8. Elément jetable selon la revendication 7, **caractérisé en ce que** la lancette (4) comprend un capuchon protecteur de pointe (7) en matière plastique, lequel est relié au corps de lancette (31) par un deuxième point destiné à la rupture (53) et.en ce que les deux points destinés à la rupture sont séparables après la mise en place de l'élément jetable (3).

9. Elément jetable selon l'une des revendications 7 ou 8, **caractérisé en ce qu'**il est réalisé de telle façon que le premier point destiné à la rupture (52) et le deuxième point destiné à la rupture (53) sont séparables en une seule manipulation.

10. Elément jetable selon la revendication 9, **caractérisé en ce que** le capuchon protecteur de pointe (7) est relié à la pièce de contact cutané (5) au niveau du bord de l'orifice de sortie (6) et **en ce que** le premier point destiné à la rupture (52) est prévu au niveau de cette liaison.

11. Elément jetable selon l'une des revendications 7 à 10, **caractérisé en ce que** la pièce de contact cutané (5) est disposée avant le corps de lancette (31) dans le sens d'introduction et **en ce que** le capuchon protecteur de pointe (7) s'étend à travers l'orifice de sortie (6) de la pièce de contact cutané (5).

12. Elément jetable selon l'une des revendications 7 à 11, **caractérisé en ce qu'**il est une pièce moulée par injection en matière plastique, à l'exception de l'aiguille de lancette réalisée en métal.
